(19) Europäisches Patentamt / European Patent Office / Office européen des brevets

(11) **EP 2 244 696 B1**

(12) **EUROPÄISCHE PATENTSCHRIFT**

(45) Veröffentlichungstag und Bekanntmachung des Hinweises auf die Patenterteilung:
**05.08.2015 Patentblatt 2015/32**

(21) Anmeldenummer: **09702374.1**

(22) Anmeldetag: **12.01.2009**

(51) Int Cl.:
*A61K 9/16* *(2006.01)*

(86) Internationale Anmeldenummer:
**PCT/EP2009/000124**

(87) Internationale Veröffentlichungsnummer:
**WO 2009/090027 (23.07.2009 Gazette 2009/30)**

(54) **TRÄGERPELLETS, VERFAHREN ZU DEREN HERSTELLUNG UND DEREN VERWENDUNG**

CARRIER PELLETS, METHOD FOR THE PRODUCTION THEREOF AND USE THEREOF

SUPPORT SOUS FORME DE PASTILLES, LEURS PROCÉDÉS DE PRODUCTION ET LEUR UTILISATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **17.01.2008 DE 102008004893**

(43) Veröffentlichungstag der Anmeldung:
**03.11.2010 Patentblatt 2010/44**

(60) Teilanmeldung:
**15172814.4**

(73) Patentinhaber: **ADD Advanced Drug Delivery Technologies, Ltd.**
**4133 Pratteln (CH)**

(72) Erfinder:
• **WEIGT, Antje**
**01277 Dresden (DE)**
• **KEMPE, Wolfgang**
**01169 Dresden (DE)**
• **SCHLÜTERMANN, Burkhard**
**79280 Au (DE)**

(74) Vertreter: **Pfenning, Meinig & Partner GbR**
**Patent- und Rechtsanwälte**
**Theresienhöhe 11a**
**80339 München (DE)**

(56) Entgegenhaltungen:
| | |
|---|---|
| EP-A1- 1 262 198 | EP-A1- 1 317 925 |
| EP-A2- 1 958 520 | WO-A1-97/16078 |
| WO-A2-2006/052503 | DE-A1- 19 733 094 |

JP-A- 2001 294 524   US-A- 5 902 844
US-A1- 2005 163 855   US-B1- 6 492 024

• **ONEDA F ET AL: "The effect of formulation variables on the dissolution and physical properties of spray-dried microspheres containing organic salts" POWDER TECHNOLOGY, ELSEVIER SEQUOIA, LAUSANNE, CH, Nr. 130, 1. Januar 2003 (2003-01-01), Seiten 377-384, XP002429927 ISSN: 0032-5910**
• **DATABASE CA CHEMICAL ABSTRACTS SERVICE, COLUMBUS, OHIO, US; 6. Dezember 2007 (2007-12-06), XP002555058 Database accession no. 147:528145 & JP 2007 297313 A (LION CORP) 15. November 2007 (2007-11-15)**
• **JACOB ET AL: "ProCell technology: Modelling and application" POWDER TECHNOLOGY, ELSEVIER SEQUOIA, LAUSANNE, CH, Bd. 189, Nr. 2, 31. Januar 2009 (2009-01-31), Seiten 332-342, XP025884765 ISSN: 0032-5910 [gefunden am 2008-04-24]**
• **KORAKIANITI E S ET AL: "Sequential optimization of a pelletization process in a fluid bed rotor granulator" JOURNAL OF DRUG DELIVERY SCIENCE AND TECHNOLOGY, ED. DE SANTÉ, FR, Bd. 14, Nr. 3, 1. Mai 2004 (2004-05-01), Seiten 207-214, XP008114543 ISSN: 1773-2247**

- **NISHIKAWA G ET AL: 'LARGE SCALE UREA GRANULATION PLANTS BASED ON TEC TECHNOLOGY', [Online] 01 Januar 2001, Seiten 1 - 12, XP001562285 20010101 Gefunden im Internet: <URL:http://www.toyo-eng.co.jp/en/ advantage /technology/petrochemistry/urea_ technology/ technical_paper/pdf/2001_Large_ Scale_Urea_G ranulation_Plants.pdf>**

Bemerkungen:

Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung von Trägerpellets für arzneiliche Wirkstoffe. Ebenso betrifft die Erfindung derartige Trägerpellets, sowie diese enthaltende pharmazeutische Formulierungen. Verwendung finden die erfindungsgemäßen Trägerpellets für den Transport und die Freisetzung von arzneilichen Wirkstoffen, insbesondere im menschlichen Körper.

Pharmazeutische, insbesondere per os anwendbare Darreichungsformen sollen für die jeweilige Anwendung in geeigneter Weise formuliert sein, um eine Freisetzung der pharmazeutischen Wirkstoffe zum richtigen Zeitpunkt und ohne störende Nebeneffekte zu bewirken. So sollen beispielsweise oral verabreichbare Wirkstoffe möglichst so freigesetzt werden, dass ein unangenehmer, z.B. bitterer Geschmack im Mund vermieden wird, da dieser insbesondere bei Kindern zu Abwehrreaktionen führen kann. Andererseits müssen die Wirkstoffe im Magen oder Darm möglichst vollständig und in raschresorbierbarer Form freigesetzt werden, wenn eine systemische Behandlung angestrebt wird.

Bei der oralen Verabreichung von Arzneimitteln wird der Wirkstoff im Magen-Darm-Trakt freigesetzt und ein Teil des Wirkstoffs resorbiert. Durch Steuerung der Freisetzung des Wirkstoffs kann das Ausmaß der Resorption und die Wirkungsdauer beeinflusst werden. Entsprechend sind verschiedene Vorschläge gemacht worden, um die Wirkstofffreisetzung durch geeignete galenische Formulierungen des Wirkstoffs zu steuern.

Ein Ansatz besteht darin, Darreichungsformen mit Überzügen zu versehen, wobei die Wirkstofffreisetzung abhängig von der Löslichkeit oder Durchlässigkeit der Überzüge beeinflusst werden kann. Derartige Überzüge können beispielsweise auf Tabletten oder Kapseln aufgebracht werden. In diesem Fall besteht jedoch ein Nachteil darin, dass ein fehlerhafter oder beschädigter Überzug dazu führen kann, dass die Freisetzung der gesamten Wirkstoffdosis nicht in der gewünschten Weise gesteuert wird.

Als Alternative bieten sich multipartikuläre Darreichungsformen an, bei denen die Gesamtmenge des Wirkstoffs auf eine größere Anzahl kleinerer Einheiten, wie Pellets, verteilt ist. Werden die einzelnen Pellets mit Überzügen versehen, so unterliegt im Fall eines fehlerhaften Überzugs bei einem Pellet nur ein entsprechend geringer Anteil der gesamten Wirkstoffdosis nicht der gewünschten Freisetzung.

Ein weiterer Vorteil derartiger Darreichungsformen auf der Basis von Pellets besteht darin, dass hinreichend kleine Pellets nach Einnahme relativ rasch aus dem Magen in den Darm übertreten. Tabletten hingegen können, sofern sie nicht zerfallen, auch für längere Zeit im Magen verbleiben, wobei die Zeit zudem recht variabel ist.

Bekannte Darreichungsformen mit kontrollierter Freisetzung sind somit nicht völlig zufrieden stellend. Zudem besteht das Problem, dass sich gewünschte (vorgegebene) Freisetzungsprofile in der Regel nicht einstellen lassen. Ferner ist die Herstellung von Darreichungsformen mit kontrollierter Freisetzung oftmals schwierig. Die EP 1 262 198 A1 offenbart pharmazeutische Zusammensetzungen in der Form von granulären Pellets, die einen Wirkstoff und einen geringen Anteil an einer organischen Säure enthalten, wobei Wirkstoff und organische Säure in einer hydrophilen Matrix als Bindemittel eingebettet sind. Der Gesamtanteil an organischer Säure ist jedoch gering, sodass sich nur ein geringer Einfluss auf das Freisetzungsmilieu des Wirkstoffs ergibt.

Es besteht somit ein Bedarf an neuen Darreichungsformen mit kontrollierter Freisetzung sowie auch an neuen Verfahren zur Herstellung von Darreichungsformen mit kontrollierter Freisetzung.

Es war daher Aufgabe der vorliegenden Erfindung, Trägerpellets und ein Verfahren zu deren Herstellung bereitzustellen, die eine kontrollierte Freisetzung des beladenen, arzneilich wirksamen Bestandteils ermöglichen und die Nachteile der aus dem Stand der Technik bekannten Systeme nicht aufweisen.

Diese Aufgabe wird durch das Verfahren mit den Merkmalen des Anspruchs 1, die Trägerpellets mit den Merkmalen des Anspruchs 9 und die pharmazeutische Formulierung mit den Merkmalen des Anspruchs 10 gelöst. Die weiteren abhängigen Ansprüche zeigen vorteilhafte Weiterbildungen auf. Anspruch 11 gibt eine erfindungsgemäße Verwendung an.

Erfindungsgemäß wird ein Verfahren zur Herstellung von Trägerpellets für einen Wirkstoff bereitgestellt, bei dem

    a) eine flüssige Formulierung durch Lösen und/oder Dispergieren von mindestens einem physiologisch verträglichen pH-Regulator und mindestens einem physiologisch verträglichen Bindemittel in mindestens einem Lösungsmittel, das aus Wasser und/oder einem organischen Lösungsmittel ausgewählt ist, hergestellt wird,

    b) die flüssige Formulierung mittels Düsen in eine Wirbelschicht- oder Strahlschicht-Anlage eingebracht wird,

    c) durch Sprühgranulation in der Anlage, bei der mittels eines Trocknungsgasstroms das Lösungsmittel verdampft wird, im wesentlichen sphärische Trägerpellets gebildet werden und

    d) die Trägerpellets aus der Anlage kontinuierlich ausgetragen werden.

Es ist bevorzugt, dass.ein pH-Regulator eingesetzt wird, der im physiologischen Umfeld dahingehend regulierend wirkt, dass der pH-Wert erniedrigt bzw. erhöht wird und damit die Bioverfügbarkeit von arzneilich wirksamen Bestandteilen ermöglicht oder verstärkt wird. Dies kann aber auch dadurch erreicht werden, dass der pH-Regulator eine stabilisierende Funktion, z.B. beim Einsatz eines Puffersystems als pH-Regulator, aufweist.

Vorzugsweise ist der pH-Regulator eine organische Säure, wobei diese besonders bevorzugt ausgewählt ist aus der Gruppe bestehend aus $C_1$-$C_{18}$-Mono-, Di- und Tricarbonsäuren sowie deren Mischungen. Beispielhafte Vertreter dieser Gruppe sind Zitronensäure, Bernsteinsäure, Apfelsäure, Fumarsäure, Weinsäure, Sorbinsäure, Adipinsäure, deren Salze und Mischungen.

Eine weitere bevorzugte Ausführungsform sieht vor, dass der mindestens eine pH-Regulator ein basisches anorganisches Salz ist.

Ist der pH-Regulator ein Puffersystem, so besteht dieses aus einem sauren bzw. basischen Salz zusammen mit einer korrespondierenden Lauge oder Säure. Beispiele hierfür sind Zitronensäure/Zitrat oder Weinsäure/Tartrat.

In einer weiteren bevorzugten Variante ist der pH-Regulator eine organische Base, z.B. eine Purinbase oder eine Pyrimidinbase, oder eine Mischung dieser Basen. Die Purinbase ist bevorzugt ausgewählt aus der Gruppe bestehend aus Adenin, Guanin, Hypoxanthin, Xanthin und Mischungen hiervon. Die Pyrimidinbase ist vorzugsweise ausgewählt aus der Gruppe bestehend aus Cytosin, Uracil, Thymin und Mischungen hiervon.

Im Falle, dass der pH-Regulator ein basisches anorganisches Salz ist, ist dieses vorzugsweise ausgewählt aus der Gruppe bestehend aus $NaHCO_3$, $K_2CO_3$, $Na_2CO_3$, $KHCO_3$, $Ca(OH)_2$, $CaO$, Phosphaten sowie Mischungen hiervon.

Erfindungsgemäß enthält die Formulierung weiterhin mindestens ein physiologisch verträgliches Bindemittel. Dieses Bindemittel ist dabei vorzugsweise ausgewählt aus der Gruppe bestehend aus Methylcellulosen, Hydroxymethylcellulosen, Hydroxypropylmethylcellulosen, Alginaten, Pektinen, Polyvinylpyrrolidonen, Xanthanen sowie anderen Hydrokolloiden und Mischungen hiervon. Als Lösungsmittel werden Wasser oder organische Lösungsmittel verwendet. Als organische Lösungsmittel sind besonders bevorzugt Ethylalkohol, Isopropanol, n-Propanol oder deren Mischungen.

Das Mengenverhältnis von pH-Regulator zu Bindemittel in der flüssigen Formulierung liegt vorzugsweise im Bereich von 50:50 bis 99:1. Die erfindungsgemäße flüssige Formulierung weist 30 bis 80 Gew.-% des mindestens einen pH-Regulators, 0,5 bis 5 Gew.-% des mindestens einen Bindemittels und 15 bis 69,5 Gew.-% des mindestens einen Lösungsmittels auf.

Die Sprühgranulation kann sowohl in einer Wirbelschichtanlage als auch in einer Strahlschichtanlage erfolgen. Die Temperatur in diesen Anlagen liegt dabei vorzugsweise im Bereich von 5 bis 100 °C. Der in die Beschichtungsanlage eintretende Trocknungsgasstrom weist am Eintritt in die Anlage vorzugsweise eine Temperatur im Bereich von 5 bis 120 °C auf. Als Trocknungsgas kommen insbesondere konditionierte Luft, Stickstoff oder Inertgase, z.B. Edelgase, in Frage.

Erfolgt die Sprühgranulation in einer Wirbelschichtanlage, wird das Trocknungsgas über einen Siebboden zugeführt. Gleichzeitig wird durch oberhalb des Siebbodens angeordnete Düsen die flüssige Formulierung in die Anlage eingebracht.

Erfolgt die Sprühgranulation in einer Strahlschichtanlage, so wird das Trocknungsgas durch am Boden befindliche Längsspalte zugeführt. Die flüssige Formulierung wird über mindestens eine zwischen den Längsspalten angeordnete Düse eingebracht.

Vorzugsweise erfolgt die Einbringung der flüssigen Formulierung durch die Düse von unten nach oben.

Erfindungsgemäß werden ebenso Trägerpellets bereitgestellt, die mindestens einen physiologisch verträglichen pH-Regulator enthalten. Diese Trägerpellets werden nach dem zuvor beschriebenen Verfahren hergestellt.

Die Trägerpellets weisen einen Durchmesser im Bereich von 50 $\mu$m bis 1,5 mm, insbesondere von 90 $\mu$m bis 1,2 mm auf.

Die Trägerpellets sind dabei im Wesentlichen sphärisch. Die Trägerpellets weisen dabei eine Sphärizität von 0,8 bis 1,0, insbesondere von 0,9 bis 1,0 auf.

Die Sphärizität berechnet sich dabei nach der folgenden Formel:

$$SPHT = \frac{4\pi A}{U^2}$$

mit A = Fläche und U = Umfang.

Die Sphärizität kann mit Geräten zur Partikelgrößen- und Partikelformanalyse mit dynamischer Bildanalyse durchgeführt werden. Ein hierfür geeignetes Gerät ist beispielsweise der CHAMSIZER von Retsch-Technology.

Weiterhin ist es bevorzugt, dass das Verhältnis von Breite zu Länge der Trägerpellets im Bereich von 0,8 bis 1,0, insbesondere von 0,9 bis 1,0 liegt. Das Verhältnis Breite zu Länge berechnet sich dabei nach der folgenden Formel:

$$b/l = \frac{\min(x_C)}{\max(x_{Fe})}$$

mit $X_{Fe}$ = Feret-Durchmesser und $X_C$ = maximale Breite des Partikels.

Auch das Breite-Länge-Verhältnis lässt sich z.B. mit dem genannten CAMSIZER bestimmen.

Vorzugsweise handelt es sich bei den erfindungsgemäßen Trägerpellets um dichte Trägerpellets, was eine Gewichtsreduzierung gegenüber Extrusionspellets bedeutet.

Die Trägerpellets weisen im Wesentlichen die gleiche Partikelgröße auf, d.h. es liegt ein enger Streubereich hinsichtlich der Partikelgröße vor.

Erfindungsgemäß enthalten die Trägerpellets mindestens ein physiologisch verträgliches Bindemittel. Dieses Bindemittel ist dabei vorzugsweise ausgewählt aus der Gruppe bestehend aus Methylcellulosen, Hydroxymethylcellulosen, Hydroxypropylmethylcellulosen, Alginaten, Pektinen, Polyvinylpyrrolidonen, Xanthanen sowie anderen Hydrokolloiden sowie Mischungen hiervon.

Erfindungsgemäß wird ebenso eine pharmazeutische Formulierung enthaltend die zuvor beschriebenen Trägerpellets und mindestens einen Wirkstoff bereitgestellt.

Verwendung finden die erfindungsgemäßen Trägerpellets als Trägerstruktur für arzneilich wirksame Bestandteile.

**Beispiel 1**

**Herstellung von Dicarbonsäurepellets anhand von D/L-Apfelsäure**

1.1 Herstellen der Sprühlösung

Die Sprühlösung besteht aus gereinigtem Wasser, Methylcellulose und Apfelsäure. Aus dem gereinigten Wasser und Methylcellulose wird eine 4 %-ige Bindelösung hergestellt. Diese wird auf 70 °C temperiert. Danach erfolgt die Zugabe von Apfelsäure unter ständigem Rühren, bis eine vollständige Lösung vorliegt (Anteil gereinigtes Wasser entspricht Anteil Apfelsäure).

1.2 Teilchenausbildung

Die temperierte Sprühlösung wird in die Strahlschichtapparatur (ProCell) im Bottom Spray-Verfahren eingedüst. Es erfolgt ein stetiger Partikelaufbau durch Verdüsen der Feststofflösung im Hauptluftstrom. Dieser besteht aus zwei Teilströmen, die durch Spaltöffnungen, längs durch den Prozessraum führend, realisiert werden. Der Partikelaufbau geschieht durch das Abdampfen des Lösungsmittels Wasser, Apfelsäure und Methylcellulose verbleiben als Teilchen getrocknet im Luftstrom. Durch das definierte Strömungsprofil des Apparates trennen sich die Partikel im oberen Prozessraum aus dem zentralen Luftstrom und fließen seitlich, bedingt durch Schwerkraft und der Sogwirkung des Hauptluftstromes, zum Prozessgaseintritt zurück. Dort werden sie erneut mit dem Hauptluftstrom mitgerissen und kontinuierlich mit Feststoff aus der Sprühlösung beschichtet. Die Prozessluft ist konditioniert.

Während des kontinuierlichen Einbringens des Feststoffgemisches über Verdüsung, erfolgt gleichzeitig die Entnahme von sauren Pellets. Die Apfelsäurepellets werden für die gewünschte Korngröße fraktioniert.

Dabei kann Unterkorn und aufbereitetes Oberkorn dem Prozess zurückgeführt werden. Endprodukt ist ein homogenes, annähernd sphärisches Apfelsäurepellet mit einer gleichmäßigen Oberflächenstruktur.

**Patentansprüche**

1. Verfahren zur Herstellung von Trägerpellets für einen Wirkstoff, bei dem

   a) eine flüssige Formulierung durch Lösen und/oder Dispergieren von mindestens einem physiologisch verträglichen pH-Regulator und mindestens einem physiologisch verträglichen Bindemittel in mindestens einem Lösungsmittel, das aus Wasser und/oder einem organischen Lösungsmittel ausgewählt ist, hergestellt wird,
   b) die flüssige Formulierung mittels Düsen in eine Wirbelschicht- oder Strahlschicht-Anlage eingebracht wird,
   c) durch Sprühgranulation in der Anlage, bei der mittels eines Trocknungsgasstroms das Lösungsmittel verdampft wird, im wesentlichen sphärische Trägerpellets gebildet werden und
   d) die Trägerpellets aus der Anlage ausgetragen werden,

   **dadurch gekennzeichnet, dass** die flüssige Formulierung 30 bis 80 Gew.-% des mindestens einen pH-Regulators, 0,5 bis 5 Gew.-% des mindestens einen Bindemittels und 15 bis 69,5 Gew.-% des mindestens einen Lösungsmittels enthält und der pH-Regulator ausgewählt ist aus der Gruppe bestehend aus

   i) $C_1$-$C_{18}$-Mono-, Di- und Tricarbonsäure, sowie Mischungen hiervon;
   ii) Puffersystem aus einem sauren oder basischen Salz zusammen mit einer korrespondierenden Lauge oder Säure, wobei das Puffersystem den pH-Wert stabilisiert;
   iii) organische Base; und
   iv) basisches anorganisches Salz.

2. Verfahren nach einem der Ansprüche 1, **dadurch gekennzeichnet, dass** die $C_1$-$C_{18}$-Mono-, Di- und/oder Tricarbonsäure ausgewählt ist aus der Gruppe bestehend aus Zitronensäure, Bernsteinsäure, Apfelsäure, Fumarsäure, Weinsäure, Sorbinsäure, Adipinsäure,und Mischungen hiervon.

3. Verfahren nach einem der Ansprüche 1 oder 2, **dadurch gekennzeichnet, dass** das Puffersystem aus Zitronensäure und einem Zitrat oder aus Weinsäure und einem Tartrat besteht.

4. Verfahren nach einem der Ansprüche 1 bis 3, **dadurch gekennzeichnet, dass** die organische Base eine Purinbase oder Pyrimidinbase, oder eine Mischung von diesen, enthält oder hieraus besteht, wobei die Purinbase ausgewählt ist aus der Gruppe bestehend aus Adenin, Guanin, Hypoxanthin, Xanthin und Mischungen hiervon.

**5.** Verfahren nach einem der Ansprüche 1 bis 4, **dadurch gekennzeichnet, dass** das basische anorganische Salz $NaHCO_3$, $K_2CO_3$, $Na_2CO_3$, $KHCO_3$, $Ca(OH)_2$, CaO, Phosphate, sowie Mischungen hiervon, enthält oder hieraus besteht.

**6.** Verfahren nach einem der Ansprüche 1 bis 5, **dadurch gekennzeichnet, dass** das Bindemittel ausgewählt ist aus der Gruppe bestehend aus Methylcellulosen, Hydroxymethylcellulosen, Hydroxypropylmethylcellulosen, Alginaten, Pektinen, Polyvinylpyrrolidonen, Xanthanen sowie anderen Hydrokolloiden und Mischungen hiervon.

**7.** Verfahren nach einem der Ansprüche 1 bis 6, **dadurch gekennzeichnet, dass** als Lösungsmittel oder Emulsionsmittel Ethylalkohol, Isopropanol, n-Propanol oder Mischungen von diesen eingesetzt werden.

**8.** Verfahren nach einem der Ansprüche 1 bis 7, **dadurch gekennzeichnet, dass** die Sprühgranulation in einer Wirbelschichtanlage erfolgt, in die über einen Siebboden das Trocknungsgas zugeführt und durch oberhalb des Siebbodens angeordneten Düsen die flüssige Formulierung eingebracht wird oder dass die Sprühgranulation in einer Strahlschichtanlage erfolgt, in die über in der unteren Hälfte der Anlage angeordnete Längsspalte das Trocknungsgas zugeführt und durch mindestens eine zwischen den Längsspalten angeordnete Düse die flüssige Formulierung eingebracht wird.

**9.** Trägerpellets, erhältlich nach dem Verfahren nach einem der Ansprüche 1 bis 8, **dadurch gekennzeichnet, dass** die Trägerpellets mindestens einen physiologisch verträglichen pH-Regulator enthalten.

**10.** Pharmazeutische Formulierung enthaltend Trägerpellets nach Anspruch 9 und mindestens einen Wirkstoff.

**11.** Verwendung von Trägerpellets nach Anspruch 9 als Trägerstrukturen für arzneilich wirksame Bestandteile.

## Claims

**1.** Method for the production of pellets for an active substance, in which

a) a liquid formulation is produced by dissolving and/or dispersing at least one physiologically well-tolerated pH regulator and at least one physiologically well-tolerated binder in at least one solvent which is selected from water and/or an organic solvent,

b) the liquid formulation is introduced by means of nozzles into a fluidised bed- or spouted bed unit,

c) essentially spherical carrier pellets are formed by spray granulation in the unit in which the solvent is evaporated by means of a drying gas flow and

d) the carrier pellets are discharged from the unit,

**characterized in that** the liquid formulation contains 30 to 80% by weight of the at least one pH regulator, 0.5 to 5% by weight of the at least one binder and 15 to 69.5% by weight of the at least one solvent and wherein the pH regulator is selected from the group consisting of

i) $C_1$-$C_{18}$ mono-, di- and tricarboxylic acid and also mixtures hereof;
ii) buffer system comprising an acidic or basic salt together with a corresponding caustic solution or acid, wherein the buffer system stabilises the pH value;
iii) organic base; and
iv) basic inorganic salt.

**2.** Method according to one of claims 1, **characterised in that** the $C_1$-$C_{18}$ mono-, di- and/or tricarboxylic acid is selected from the group consisting of citric acid, succinic acid, malic acid, fumaric acid, tartaric acid, sorbic acid, adipinic acid, and mixtures hereof.

**3.** Method according to claim 1 or 2, **characterised in that** the buffer system comprises citric acid and a citrate or tartaric acid and a tartrate.

**4.** Method according to one of the claims 1 to 3, **characterised in that** the organic base comprises or consists of a purine base or a pyrimidine base, or a mixture of these, wherein the purine base is selected from the group consisting of adenine, guanine, hypoxanthine, xanthine and mixtures hereof.

**5.** Method according to one of the claims 1 to 4, **characterized in that** the basic inorganic salt comprises or consists of $NaHCO_3$, $K_2CO_3$, $Na_2CO_3$, $KHCO_3$, $Ca(OH)_2$, CaO, phosphates and mixtures hereof.

**6.** Method according to one of the claims 1 to 5, **characterised in that** the binder is selected from the group consisting of methyl celluloses, hydroxymethyl celluloses, hydroxypropylmethyl celluloses, alginates, pectins, polyvinylpyrrolidones, xanthanes and also other hydrocolloids and mixtures hereof.

**7.** Method according to one of the claims 1 to 6, **characterised in that** ethyl alcohol, isopropanol, n-propanol or mixtures of these, is used as solvent or emulsifier.

**8.** Method according to one of the claims 1 to 7, **characterised in that** the spray granulation is effected in a fluidised bed unit into which the drying gas is supplied via a sieve plate and the liquid formulation is introduced by nozzles disposed above the sieve plate or that the spray granulation is effected in a spouted bed unit into which the drying gas is supplied via a longitudinal gap disposed in the lower half of the unit and the liquid formulation is introduced by at least one nozzle disposed between the longitudinal gaps.

**9.** Carrier pellets obtainable according to the method according to one of the claims 1 to 8, **characterized in that** the carrier pellets comprise at least one physiologically well-tolerated pH regulator.

**10.** Pharmaceutical formulation comprising carrier pellets according to claim 9 and at least one active substance.

**11.** Use of carrier pellets according to claim 9 as carrier structures for pharmaceutically effective components.

**Revendications**

**1.** Procédé de fabrication de granulés formant support pour un principe actif, dans lequel

   a) on fabrique une formulation liquide par dissolution et/ou dispersion d'au moins un régulateur de pH physiologiquement compatible et d'au moins un liant physiologiquement compatible dans au moins un solvant qui est choisi parmi l'eau et/ou un solvant organique,
   b) on introduit la formulation liquide par des buses dans une installation à lit fluidisé ou à lit jaillissant,
   c) par granulation par atomisation dans l'installation au cours de laquelle le solvant est évaporé grâce à un courant des gaz de séchage, on forme des granulés formant support, sensiblement sphériques, et
   d) on expulse de l'installation les granulés formant support,

   **caractérisé en ce que** la formulation liquide contient 30 à 80 % en poids du ou des régulateurs de pH, 0,5 à 5 % en poids du ou des liants et 15 à 69,5 % en poids du ou des solvants, et que le régulateur de pH est choisi dans le groupe consistant en :

   i) un acide mono-, di- ou tricarboxylique en $C_1$-$C_{18}$, ainsi que les mélanges de ceux-ci,

   ii) un système tampon constitué d'un sel acide ou basique conjointement à une base ou à un acide correspondant, le système tampon stabilisant le pH,
   iii) une base organique ; et
   iv) un sel inorganique basique.

**2.** Procédé selon la revendication 1, **caractérisé en ce que** l'acide mono-, di- et/ou tricarboxylique en $C_1$-$C_{18}$ est choisi dans le groupe consistant en l'acide citrique, l'acide succinique, l'acide malique, l'acide fumarique, l'acide tartrique, l'acide sorbique, l'acide adipique et les mélanges de ceux-ci.

**3.** Procédé selon l'une des revendications 1 ou 2, **caractérisé en ce que** le système tampon est constitué d'acide citrique et d'un citrate, ou d'acide tartrique et d'un tartrate.

**4.** Procédé selon l'une des revendications 1 à 3, **caractérisé en ce que** la base organique contient une base purique ou une base pyrimidique, ou un mélange de celles-ci, ou en est constituée, la base purique étant choisie dans le groupe consistant en l'adénine, la guanine, l'hypoxanthine, la xanthine et les mélanges de celles-ci.

**5.** Procédé selon l'une des revendications 1 à 4, **caractérisé en ce que** le sel inorganique basique contient $NaHCO_3$, $K_2CO_3$, $Na_2CO_3$, $KHCO_3$, $Ca(OH)_2$, CaO, des phosphates, ainsi que des mélanges de ceux-ci, ou en est constitué.

**6.** Procédé selon l'une des revendications 1 à 5, **caractérisé en ce que** le liant est choisi dans le groupe consistant en les méthylcelluloses, les hydroxyméthylcelluloses, les hydroxypropylméthylcelluloses, les alginates, les pectines, les polyvinylpyrrolidones, les xanthanes, ainsi que d'autres hydrocolloïdes, et les mélanges de ceux-ci.

**7.** Procédé selon l'une des revendications 1 à 6, **caractérisé en ce qu'**on utilise en tant que solvant ou émulsifiant de l'alcool éthylique, de l'isopropanol, du n-propanol ou des mélanges de ceux-ci.

**8.** Procédé selon l'une des revendications 1 à 7, **caractérisé en ce que** la granulation par atomisation a lieu dans une installation à lit jaillissant, dans laquelle le gaz de séchage est introduit par l'intermédiaire d'un fond perforé, et la formulation liquide est introduite par des buses disposées au-dessus du fond perforé, et **en ce que** la granulation par atomisation a lieu dans une installation à lit jaillissant dans laquelle le gaz de séchage est amené par une fente longitudinale disposée dans la moitié inférieure de l'installation, la formulation liquide étant introduite par au moins une buse disposée entre les fentes longitudinales.

9. Granulés formant support pouvant être obtenus par le procédé selon l'une des revendications 1 à 8, **caractérisés en ce que** les granulés formant support contiennent au moins un régulateur de pH physiologiquement compatible.

10. Formulation pharmaceutique contenant des granulés formant support selon la revendication 9 et au moins un principe actif.

11. Utilisation de granulés formant support selon la revendication 9 en tant que structures formant support pour constituants à effet médicamenteux.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1262198 A1